# FASCICULE DE BREVET EUROPEEN

(11) **EP 0 736 044 B1**
(45) Date de publication et mention de la délivrance du brevet: **06.05.1998**
(21) Numéro de dépôt: 95904577.4
(22) Date de dépôt: 21.12.1994
(51) Int. Cl.: C08B 37/16, A61K 47/48

(54) **DERIVES DE CYCLODEXTRINE, UTILISABLES POUR SOLUBILISER DES COMPOSES CHIMIQUES HYDROPHOBES TELS QUE DES MEDICAMENTS, ET LEURS PROCEDES DE PREPARATION**
CYCLODEXTRINDERIVATE ZUR AUFLöSUNG VON HYDROPHOBEN, CHEMISCHEN VERBINDUNGEN WIE ARZNEIMITTELN; VERFAHREN ZU DEREN HERSTELLUNG
CYCLODEXTRIN DERIVATIVES FOR SOLUBILISING HYDROPHOBIC CHEMICAL COMPOUNDS SUCH AS DRUGS, AND METHODS FOR PREPARING SAME

(30) Priorité: 22.12.1993 FR 9315472
(43) Date de publication de la demande: 09.10.1996
(73) Titulaire: COMMISSARIAT A L'ENERGIE ATOMIQUE, 75015 Paris (FR)
(72) Inventeur: DJEDAINI-PILARD, Florence, F-91150 Etampes (FR); AZAROUAL-BELLANGER, Nathalie, F-91940 Les Ulis (FR); PERLY, Bruno, F-78320 Le Mesnil-Saint-Denis (FR)
(74) Mandataire: Des Termes, Monique
(86) Numéro de dépôt international: FR9401501
(87) Numéro de publication internationale: WO9517432

(56) Documents cités:
- WO-A-91/13100
- CHINESE CHEMICAL LETTERS, vol.2, no.3, 1991, CHINA pages 205 - 208 B. J. SHEN ET AL. 'Synthesis and characterization of novel multifunctional host compounds. 1. .beta.-cyclodextrin derivative bearing diethanolamine moiety'
- DATABASE WPI Week 8728, Derwent Publications Ltd., London, GB; AN 87-194684 & JP,A,62 123 196 (NISSHIN FLOUR MILL KK) 4 Juin 1987
- CHEMICAL ABSTRACTS, vol. 82, no. 1, 6 Janvier 1975, Columbus, Ohio, US; abstract no. 4533s, page 396 ;colonne R ; & JP,A,7 485 015 (TANABE SEIYAKU CO. LTD) 15 Août 1974

## Description

La présente invention a pour objet de nouveaux dérivés de cyclodextrines, utilisables en particulier pour solubiliser dans un milieu aqueux des composés chimiques hydrophobes, tels que des molécules pharmaceutiquement actives, par inclusion dans ces dérivés.

Les cyclodextrines ou cyclomaltooligosaccharides sont des composés d'origine naturelle formés par l'enchaînement de 6, 7 ou 8 unités glucose liées en α-1,4. De nombreux travaux ont montré que ces cyclodextrines pouvaient former des complexes d'inclusion avec des molécules hydrophobes et permettre ainsi la solubilisation de ces molécules dans des milieux aqueux. De nombreuses applications ont été proposées pour tirer profit de ce phénomène, en particulier dans le domaine pharmaceutique, comme il est décrit par D. Duchêne dans l'ouvrage intitulé "Cyclodextrins and their industrial uses", chapitre 6, pages 213 à 257, Editions de Santé, 1987. Des compositions pharmaceutiques utilisant ces cyclodextrines ont d'ailleurs été commercialisées au Japon, en Italie et plus récemment en France, par exemple par Pierre Fabre Médicament pour le Brexin® qui est un complexe d'inclusion du Piroxicam dans la β-cyclodextrine.

Parmi les cyclodextrines utilisables, la β -cyclodextrine qui comporte 7 unités glucose, est la plus adaptée au niveau de la taille de sa cavité et la moins chère des trois, mais son utilisation pose certains problèmes, car elle est moins soluble que les autres cyclodextrines et présente un caractère hémolytique.

Aussi, on a envisagé d'améliorer les propriétés de la β-cyclodextrine en la modifiant chimiquement pour la rendre plus adaptée. Plusieurs solutions ont été envisagées et ont conduit à l'utilisation de dérivés méthylés ou de dérivés hydroxyalkylés.

Les dérivés méthylés sont beaucoup plus solubles que la cyclodextrine d'origine et ils possèdent de bonnes propriétés de solubilisation de composés organiques hydrophobes, en particulier dans le cas de la 2,6-diméthyl-β-cyclodextrine. Néanmoins, ces dérivés méthylés, outre qu'ils sont difficiles à obtenir à l'état pur, sont inutilisables pour des applications pharmaceutiques, en particulier pour les formes injectables, en raison de leur très fort caractère hémolytique.

Les dérivés hydroxyalkylés, développés en particulier par Janssen, par exemple les hydroxypropyl-cyclodextrines décrites par L. SZENTE et C.E. STRATTAN, « hydroxypropyl-β-cyclodextrins : Preparation and Physico-Chemical Properties », New Trends in Cyclodextrins and Derivatives, D. Duchêne Ed., Editions de Santé, Paris, 1991, chapitre 2, pp 57-96, présentent une très forte solubilité dans l'eau et sont peu hémolytiques. Toutefois, leur utilisation reste difficile en raison de leur extrême hétérogénéité chimique ; de plus, les substitutions peuvent limiter la formation de complexes d'inclusion par effet de gêne stérique, si bien qu'aucune application pharmaceutique n'a encore été mise au point avec ces dérivés.

On a envisagé plus récemment d'utiliser d'autres dérivés des cyclodextrines, comme il est décrit dans WO-A-91/13100. Parmi ces dérivés, on trouve des dérivés substitués par une diamine, mais les résultats obtenus avec ces dérivés diamino ne donnent pas entièrement satisfaction pour des applications pharmaceutiques.

La présente invention a précisément pour objet de nouveaux dérivés de cyclodextrines, utilisables pour solubiliser des composés chimiques hydrophobes, qui pallient ces inconvénients.

Ces nouveaux dérivés de cyclodextrines répondent à la formule : 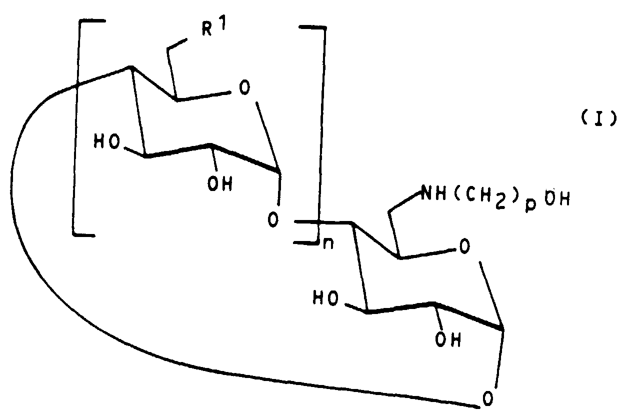 dans laquelle R¹ représente OH ou NH (CH₂)ₚOH, n est égal à 5,6 ou 7, et p est un nombre entier allant de 2 à 6, les p, pouvant être différents lorsque un ou plusieurs des R¹ représentent NH(CH₂)ₚOH.

Selon un premier mode de réalisation de l'invention, le dérivé de cyclodextrine est un dérivé monosubstitué, c'est-à-dire que dans la formule I précitée, tous les R¹ représentent OH.

Un tel dérivé peut être préparé en faisant réagir un dérivé tosylé de formule : 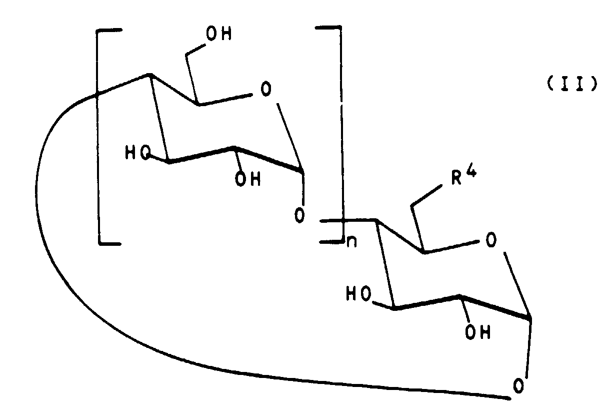 dans laquelle R⁴ représente le groupe tosyle et n est égal à 5, 6 ou 7, avec un composé de formule NH₂ (CH₂)ₚOH avec p ayant la signification donnée ci-dessus.

Le dérivé tosylé de formule (II) peut être obtenu par exemple par le procédé décrit dans J.Am. Chem. Soc., 112, 1990, page 3860-3868.

La réaction du dérivé tosylé avec le composé de formule NH₂ (CH₂)ₚOH peut être effectuée simplement par dissolution du dérivé tosylé dans ce composé.

Selon un second mode de réalisation de l'invention, le dérivé de cyclodextrine est un dérivé persubstitué, c'est-à-dire répondant à la formule I précitée dans laquelle tous les R¹ représentent un groupe NH(CH₂)ₚOH avec p ayant la signification donnée ci-dessus.

Ces dérivés persubstitués sont particulièrement intéressants, car la présence de plusieurs groupements NH (CH²)ₚOH permet d'augmenter fortement leur solubilité dans l'eau.

Ces dérivés peuvent être préparés à partir des dérivés periodés de cyclodextrines par réaction d'un , dérivé iodé de cyclodextrine répondant à la formule : 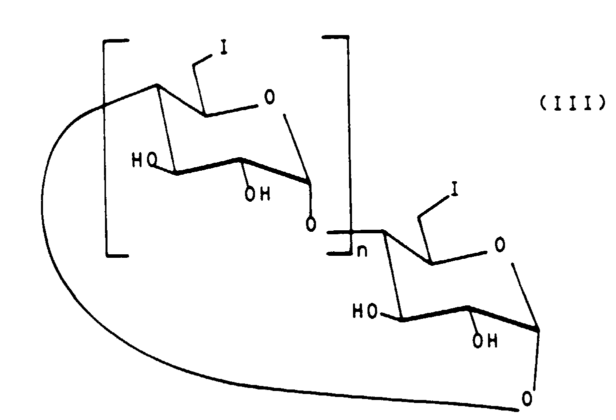 dans laquelle n est égal à 5, 6 ou 7 avec au moins un composé de formule NH₂(CH₂)ₚOH dans laquelle p a la signification donnée ci-dessus.

De préférence, dans ce second mode de réalisation de l'invention tous les R¹ du dérivé de formule (I) sont identiques.

Dans ce cas, on utilise un seul composé de formule NH₂ (CH₂)ₚOH pour obtenir le dérivé persubstitué.

Les dérivés de cyclodextrines décrits ci-dessus sont utilisables en particulier pour solubiliser en milieu aqueux des composés chimiques hydrophobes.

Aussi, l'invention a également pour objet un procédé de solubilisation dans un milieu aqueux d'un composé chimique hydrophobe, qui consiste à combiner ce composé avec un dérivé de cyclodextrine répondant à la formule (I) précitée, pour former avec celui-ci un complexe d'inclusion soluble dans l'eau.

L'utilisation dans ce procédé de dérivés répondant à la formule (I) précitée présente l'avantage d'améliorer la solubilité, la stabilité et la biodisponibilité, sous diverses formes d'administration, du composé hydrophobe, en particulier lorsqu'il s'agit de molécules pharmaceutiquement actives.

De préférence dans l'invention, on utilise les dérivés mono ou persubstitués de la β-cyclodextrine, c'est-à-dire que dans la formule I précitée,n est égal à 6. On peut toutefois aussi utiliser les dérivés de l'α-cyclodextrine (n=5) et les dérivés de la γ-cyclodextrine (n=7).

Les composés chimiques hydrophobes susceptibles d'être solubilisés dans des milieux aqueux au moyen de ces cyclodextrines peuvent être de différents types.

A titre d'exemples de tels composés, on peut citer des produits cosmétiques, des vitamines, des molécules pharmaceutiquement actives telles que celles décrites par D. Duchêne dans l'ouvrage intitulé "Cyclodextrins and their industrial uses", chapitre 6, pages 213 à 257, Editions de Santé, 1987).

De préférence dans l'invention, le composé chimique hydrophobe est une molécule pharmaceutiquement active.

A titre d'exemples de telles molécules, on peut citer les stéroïdes, par exemple la prednisolone, les agents anti-épileptiques comme la carbamazépine, et les agents anti-cancéreux.

L'invention concerne également les complexes d'inclusion des dérivés de cyclodextrines mentionnés ci-dessus avec un composé chimique, en particulier hydrophobe, tel qu'une molécule pharmaceutiquement active.

Ces complexes d'inclusion peuvent être préparés par des procédés classiques, par exemple en ajoutant à une solution ou à une suspension du dérivé de cyclodextrine de formule (I) utilisé, une solution du composé dans un solvant organique approprié, par exemple l'acétone. On peut ensuite isoler le complexe d'inclusion ainsi formé par lyophilisation.

L'invention a encore pour objet une composition pharmaceutique comprenant un complexe d'inclusion d'un dérivé de cyclodextrine de formule (I) et d'une molécule pharmaceutiqiuement active, avec un véhicule pharmaceutiquement acceptable.

Ces compositions pharmaceutiques qui peuvent être administrées par voie orale ou parentérale, sont par exemple des solutions, des poudres, des suspensions, etc., en particulier des solutions injectables.

D'autres caractéristiques et avantages de l'invention apparaîtront mieux à la lecture des exemples suivants, donnés bien entendu à titre illustratif et non limitatif.

### Exemple 1 : Préparation du mono-6-éthanolamino-6-désoxy-cyclomaltoheptaose.

Ce dérivé est le dérivé monosubstitué de formule I avec tous les R¹ représentant OH, p étant égal à 2 et n étant égal à 6.

On prépare tout d'abord le dérivé tosylé de formule II avec n=6, en opérant de la façon suivante.

60 g de cyclomaltoheptaose (52,8 mmol) sont suspendus dans 500 ml d'eau distillée. On ajoute goutte à goutte 6,57 g (164 mmol) de soude caustique dissoute dans 20 ml d'eau sur 5 minutes avec forte agitation magnétique. A la solution limpide obtenue sont ajoutés 10,08 g (52,9 mmol) de chlorure de p-toluène sulfonyle (chlorure de tosyle) dans 30 ml d'acétonitrile goutte à goutte sur 10 minutes. Après 2 h d'agitation à température ambiante, le précipité formé est éliminé par filtration et le filtrat est conservé 48 h à 4°C. Le précipité est isolé par filtration sous vide, lavé par 50 ml d'eau glacée et recristallisé immédiatement dans l'eau bouillante. Après une nuit à 4°C, le précipité est filtré et séché sous vide à 30°C. On obtient ainsi 7,5 g (12 %) d'un composé pur conforme aux spécifications.

Dans un ballon, on dissout 2 g (1,55 mmol) de 6-tosyl-6-désoxycyclomaltoheptaose obtenu précédemment dans 8 ml d'éthanolamine pure. On laisse le mélange réactionnel sous agitation pendant 3 jours à la température ambiante, puis on ajoute le mélange réactionnel goutte à goutte à 200 ml d'éthanol absolu. On maintient la suspension pendant 3 jours à 0°C, puis on filtre le précipité sous vide, et on le lave à l'éthanol absolu puis à l'acétone. On redissout le solide obtenu dans de l'eau et on lyophilise. On obtient ainsi 1,1 g de 6-éthanolamino-6-désoxy-cyclomaltoheptaose, ce qui correspond à un rendement de 60 %.

Les caractéristiques de ce composé sont les suivantes :
Chromatographie sur couches minces (Plaques de Silice Merck) Rf=0,60 dans le mélange NH₄OH 6 %/Ethanol/n-Butanol 5:5:1 (v:v), révélation par H₂SO₄ 10 %.
Spectrométrie de masse : Mode Electrospray:m/z=1178[M+H]⁺
RMN (500 MHz, solution à 10 mmol/l dans l'eau lourde)
H-1:5,12 ppm, H-6a et H-6a':2,891 et 3,15 ppm
NH-CH₂:2,8 ppm, H-4a:3,50 ppm, CH₂OH:3,64 ppm,
Autres protons de la cyclodextrine:3,6 à 3,8 et de 3,85 à 4,05 ppm.

| Analyse élémentaire C₄₄H₇₅NO₃₅, 3H₂O: | | | | |
|---|---|---|---|---|
| | C | H | N | O |
| CALCULEE | 42,89 | 6,63 | 1,14 | 49,34 |
| TROUVEE | 41,7 | 6,55 | 1,18 | 48,6 |

Solubilité dans l'eau dans des conditions physiologiques (tampon phosphate de sodium, pH 7,40, 25°C) : 40 mmol/l sous forme de base libre, et 95 mmol/l sous forme de chlorhydrate.

### Exemple 2 : Préparation de l'heptakis-6-éthanolamino-6-désoxy-cyclomaltoheptaose.

Ce dérivé est le dérivé de formule I dans laquelle tous les R¹ représentent NH-CH₂₋CH₂₋OH, p est égal à 2 et n est égal à 6.

On dissout dans 6 ml d'éthanolamine, 300 mg (0,157 mmol) d'heptakis (6-iodo-6-désoxy)cyclomaltoheoptaose et on maintient le mélange réactionnel sous agitation et sous atmosphère d'argon à la température ambiante pendant 18 heures. Après concentration sous vide à environ 1 ml, on verse la solutions goutte à goutte et sous forte agitation dans 200 ml d'acétone anhydre. On isole le précipité obtenu par filtration, on le lave par de l'acétone, puis on le sèche sous vide à 30°C. On redissout le résidu dans 2 ml d'eau, on filtre sur Millex GS (0,2 µm) et on lyophilise. On obtient ainsi 0,177 g d'heptakis-6-éthanolamino-6-désoxy-cyclomaltoheptaose, ce qui correspond à un rendement de 78 %.

Les caractéristiques de ce composé sont les suivantes :
Chromatographie sur couches minces de Silicagel Merck:Rf:0,65 dans le mélange NH₄OH 6 %/Ethanol/n-Butanol.
RMN (solution dans l'eau 20 mmol/l). Les spectres aussi bien en proton qu'en C13 indiquent une symétrie complète de la molécule et donc une persubstitution.
RMN du proton : attribution par corrélation homonucléaire à deux quanta (500 MHz, 298K) 5,153 (H-1), 3,68 (H-2), 4,0 (H-3), 3,55 (H-4), 3,99 (H-5), 2,92 et 3,05 (H-6_{ab}), 2,8 (NH-C**H**_{**2**}-CH₂-OH), 3,71 et 3,74 (NH-CH₂-C**H**_{**2**}-OH).
RMN du Carbon 13 : attribution par corrélation C-H en deux dimensions : 102,6 (C-1), 73,5 (C-2), 74,0 (C-3), 83,9 (C-4), 71,5 (C-5), 50,3 (C-6), 51,7 (NH-**C**H₂-CH₂-OH), 61,1(NH-CH₂-**C**H₂-OH).
Solubilité dans l'eau dans des conditions physiologiques (tampon phosphate de sodium, pH 7,40, 25°C) : 140 mmol/l sous forme de base libre, et 350 mmol/l sous forme de chlorhydrate.

### Exemple 3 : Préparation d'un complexe d'heptakis-6-éthanolamino-6-désoxy-cyclomaltoheptaose et de prednisolone.

La prednisolone répond à la formule suivante : 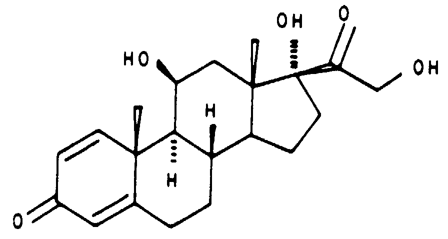 et elle a une très faible solubilité dans l'eau (0,25 mg/ml à 25°C, soit 0,7 mmol/l).

On dissout 10 µmol de l'heptakis-6-éthanolamino-6-désoxycyclomaltoheptaose (sous forme de chlorhydrate) préparé dans l'exemple 1, dans 1 ml d'eau pure (eau apyrogène pour injection) et on ajoute 5 µmol de prednisolone sous forme d'une solution concentrée à 50 mmol/l dans l'acétone. On élimine l'acétone par passage d'azote pendant 10 min. et on lyophilise la solution.

Le solide résiduel qui contient 10 µmol du dérivé de cyclodextrine et 5 µmol de prednisolone, est redissous dans le minimum d'eau à 25°C. Ce minimum correspond à 160 µl d'eau, ce qui indique une solubilité de 30 mmol/l de prednisolone dans l'eau, en présence de ce dérivé de cyclodextrine à une concentration de 60 mmol/l.

Dans les mêmes conditions, la solubilité maximale atteinte en prednisolone dans le cas du dérivé monosubstitué de l'exemple 1 est de 18 mmol/l.

Dans les mêmes conditions, la β-cyclodextrine ne permet de solubiliser la prednisolone qu'à hauteur de 9 mmol/l.

Ainsi, le dérivé persubstitué de la β-cyclodextrine conforme à l'invention permet d'atteindre un résultat bien supérieur.

A titre comparatif, dans les mêmes conditions que celles des exemples 1, 2 et 3, le dérivé de β -cyclodextrine conforme au document WO-A-91/13100 répondant à la formule (I) donnée ci-dessus, dans laquelle tous les R¹ représentent OH, n est égal à 6, et NH (CH₂)ₚOH est remplacé par NH-CH₂-CH₂-NH₂ a une solubilité maximale dans l'eau de 70 mmol/l, et ne permet de solubiliser la prednisolone qu'à hauteur de 12 mmol/l.

Les dérivés de l'invention sont donc très intéressants, car ils sont beaucoup plus solubles dans l'eau que la β-cyclodextrine dont la solubilité dans l'eau est de 15 mmol/l (18 g/l) à 25°C, alors que le chlorhydrate du dérivé persubstitué a une solubilité de 350 mmol/l.

Ils ont ainsi un fort pouvoir solubilisant pour de nombreux principes actifs.

De plus, ils ont un très faible caractère hémolytique.

En effet, les propriétés hémolytiques du dérivé obtenu dans l'exemple 1 ont été testées en mettant en contact 0,4 ml d'une suspension d'érythrocytes humains avec 4 ml de la solution du chlorhydrate à 5 mmol/l, à un pH de 7,4, pendant 30 minutes à 37°C. Dans ces conditions, on n'a observé aucune hémolyse alors que le taux d'hémolyse est de 50 % avec la β-cyclodextrine dans les mêmes conditions.

Par ailleurs, des mesures de potentiel d'ionisation (pK) réalisées par potentiométrie ont montré que ces dérivés se situent dans la zone de pouvoir tampon optimal pour les conditions physiologiques, ce qui les rend intéressants pour des applications parentérales.

Ainsi, le dérivé monosubstitué de l'exemple 1 présente un seul pK à 7,10.

Le dérivé persubstitué de l'exemple 2 présente un pK unique à 7,05.

Ainsi, dans les conditions physiologiques (pH aux environs de 7,2 à 7,4), ces dérivés se situent dans la zone de pouvoir tampon optimal (le pouvoir tampon est optimal autour de la valeur pK).

En revanche, dans le cas du dérivé de formule (I) conforme au document WO-A-91/13100 avec R¹ = OH, n = 6 et NH (CH₂)ₚOH remplacé par NH-CH₂-CH₂-NH₂, on observe deux pK à 7,5 et 9,5.

Enfin, les dérivés de l'invention sont faciles à obtenir, sous forme d'espèces chimiques uniques, parfaitement caractérisées, sans qu'il soit nécessaire de mettre en oeuvre des étapes de purification complexes.

## Revendications

1. Dérivé de cyclodextrine répondant à la formule : 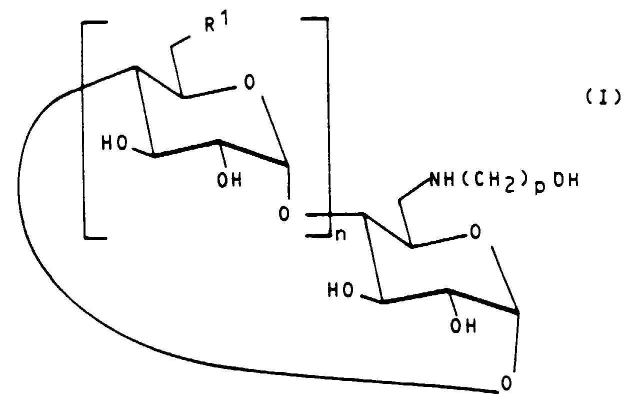 dans laquelle R¹ représente OH ou NH(CH₂)ₚOH, n est égal à 5, 6 ou 7, et p est un nombre entier allant de 2 à 6, les p pouvant être différents lorsque un ou plusieurs des R¹ représentent NH(CH₂)ₚOH.

2. Dérivé selon la revendication 1, caractérisé en ce que tous les R¹ représentent OH.

3. Dérivé selon la revendication 1, caractérisé en ce que tous les R¹ représentent NH(CH₂)ₚOH.

4. Dérivé selon la revendication 3, caractérisé en ce que tous les R₁ sont identiques.

5. Dérivé selon l'une quelconque des revendications 1 à 4, caractérisé en ce que p est égal à 2.

6. Dérivé selon l'une quelconque des revendications 1 à 5, caractérisé en ce que n est égal à 6.

7. Procédé de préparation d'un dérivé de cyclodextrine selon la revendication 2, caractérisé en ce que l'on fait réagir un dérivé tosylé de cyclodextrine de formule 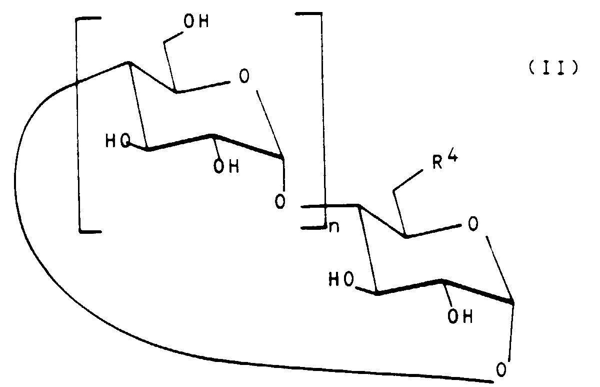 dans laquelle R⁴ représente le groupe tosyle, et n est égal à 5, 6 ou 7, avec un composé de formule NH₂(CH)₂)ₚOH avec p ayant la signification donnée dans la revendication 1.

8. Procédé de préparation d'un dérivé de cyclodextrine selon l'une quelconque des revendications 3 et 4, caractérisé en ce que l'on fait réagir un dérivé iodé de cyclodextrine de formule : 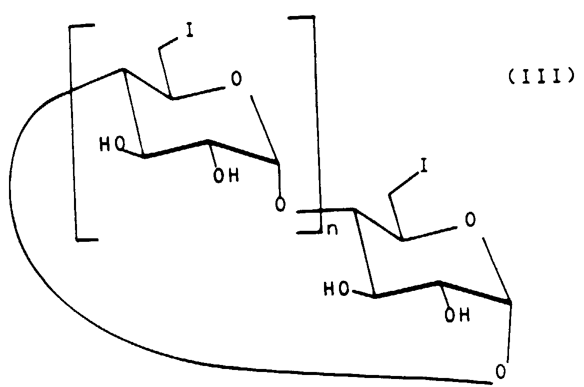 dans laquelle n est égal à 5, 6 ou 7 avec au moins un composé de formule NH₂(CH₂)ₚOH dans laquelle p a la signification donnée dans la revendication 1.

9. Procédé de solubilisation dans un milieu aqueux d'un composé chimique hydrophobe, caractérisé en ce qu'il consiste à combiner le composé chimique hydrophobe avec un dérivé de cyclodextrine selon l'une quelconque des revendications 1 à 6, pour former avec celui-ci un complexe d'inclusion soluble dans l'eau.

10. Procédé selon la revendication 9, caractérisé en ce que le composé hydrophobe est une molécule pharmaceutiquement active.

11. Complexe d'inclusion d'un dérivé de cyclodextrine selon l'une quelconque des revendications 1 à 6 avec un composé chimique hydrophobe.

12. Complexe selon la revendication 11, caractérisé en ce que le composé chimique est une molécule pharmaceutiquement active.

13. Complexe selon la revendication 12, caractérisé en ce que la molécule pharmaceutiquement active est la prednisolone.

14. Composition pharmaceutique, caractérisée en ce qu'elle comprend un complexe d'inclusion d'un dérivé de cyclodextrine selon l'une quelconque des revendications 1 à 6 et d'une molécule pharmaceutiquement active, avec un véhicule pharmaceutiquement acceptable.

## Patentansprüche

1. Cyclodextrinderivat mit der folgenden Formel: 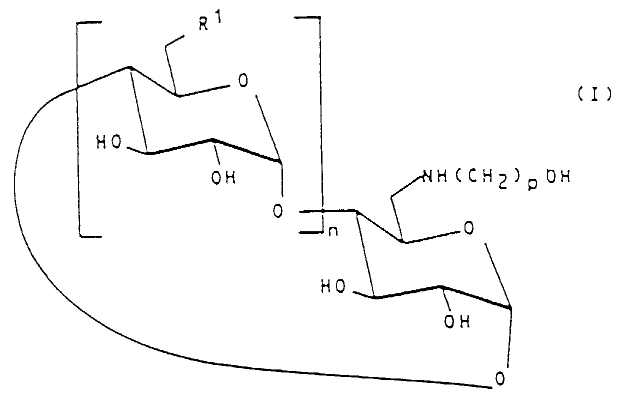 worin R¹ OH oder NH (CH₂)ₚOH bedeutet, n bedeutet 5, 6 oder 7 und p ist eine ganze Zahl von 2 bis 6, wobei die p unterschiedlich sein können, wenn ein oder mehrere von R¹ NH(CH₂)ₚOH bedeuten.

2. Derivat nach Anspruch 1, **dadurch gekennzeichnet**, daß alle R¹ OH bedeuten.

3. Derivat nach Anspruch 1, **dadurch gekennzeichnet**, daß alle R¹ NH(CH₂)ₚOH bedeuten.

4. Derivat nach Anspruch 3, **dadurch gekennzeichnet**, daß alle R¹ identisch sind.

5. Derivat nach einem oder mehreren der Ansprüche 1 bis 4, **dadurch gekennzeichnet**, daß p 2 bedeutet.

6. Derivat nach einem oder mehreren der Ansprüche 1 bis 5, **dadurch gekennzeichnet**, daß n 6 bedeutet.

7. Verfahren zur Herstellung eines Cyclodextrinderivats nach Anspruch 2, **dadurch gekennzeichnet**, daß man ein tosyliertes Derivat des Cyclodextrins der Formel 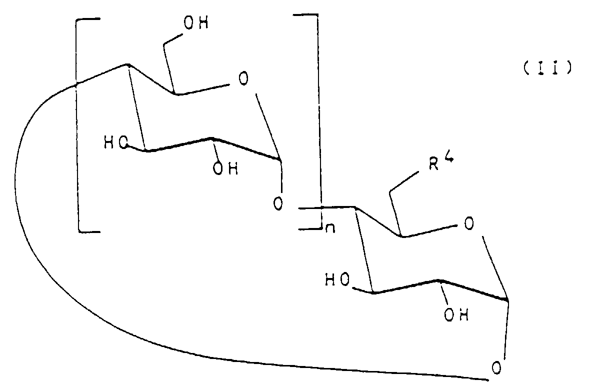 worin R⁴ die Tosylgruppe bedeutet und n 5, 6 oder 7 bedeutet, mit einer Verbindung der Formel NH₂(CH)₂ₚOH umsetzt, wobei p die in Anspruch 1 angegebene Bedeutung hat.

8. Herstellungsverfahren für ein Cyclodextrinderivat nach einem der Ansprüche 3 und 4, **dadurch gekennzeichnet**, daß man ein iodiertes Derivat des Cyclodextrins der Formel 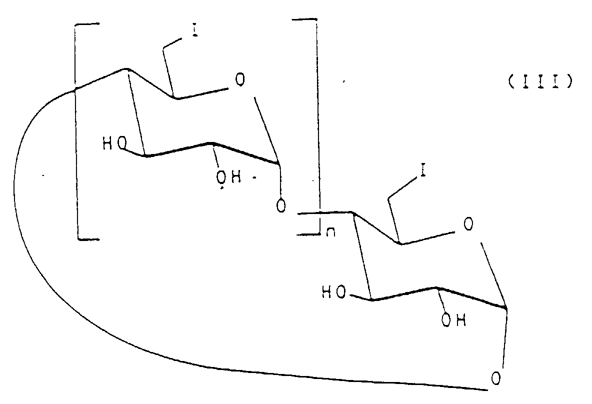 worin n 5, 6 oder 7 bedeutet mit mindestens einer Verbindung der Formel NH₂(CH₂)ₚOH umsetzt, worin p die in Anspruch 1 angegebenen Bedeutung hat.

9. Verfahren zum Lösbarmachen einer hydrophoben chemischen Verbindung in einem wäßrigen Milieu, **dadurch gekennzeichnet**, daß es den Schritt der Kombination der hydrophoben chemischen Zusammensetzung mit einem Cyclodextrinderivat nach einem oder mehreren der Ansprüche 1 bis 6 umfaßt, um mit diesem einen Einschlußkomplex zu bilden, der in Wasser löslich ist.

10. Verfahren nach Anspruch 9, **dadurch gekennzeichnet**, daß die hydrophobe Verbindung ein pharmazeutisch aktives Molekül ist.

11. Einschlußkomplex eines Cyclodextrinderivats nach einem oder mehreren der Ansprüche 1 bis 6 mit einer hydrophoben chemischen Verbindung.

12. Komplex nach Anspruch 11, **dadurch gekennzeichnet**, daß die chemische Verbindung ein pharmazeutisch aktives Molekül ist.

13. Komplex nach Anspruch 12, **dadurch gekennzeichnet**, daß das pharmazeutisch aktive Molekül Prednisolon ist.

14. Pharmazeutische Zusammensetzung, **dadurch gekennzeichnet**, daß sie einen Einschlußkomplex eines Cyclodextrinderivats nach einem oder mehreren der Ansprüche 1 bis 6 und ein pharmazeutisch aktives Molekül mit einem pharmazeutisch akzeptablen Träger umfaßt.

## Claims

1. Cyclodextrin derivative in accordance with the formula: 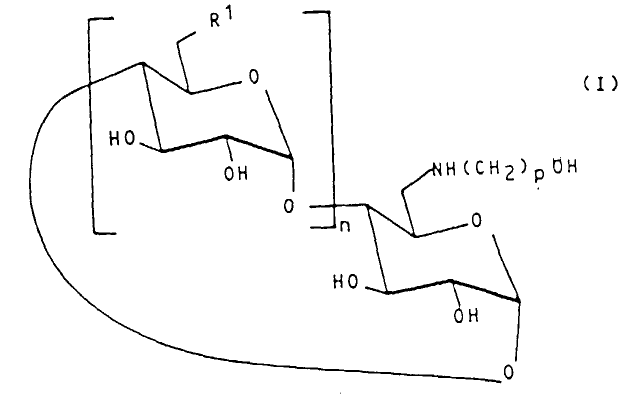 in which R¹ represents OH or NH(CH₂)ₚOH, n is equal to 5, 6 or 7 and p is an integer from 2 to 6 and the p can be different when one or more of the R¹ represent NH(CH₂)ₚOH.

2. Derivative according to claim 1, characterized in that all the R¹ represent OH.

3. Derivative according to claim 1, characterized in that all the R¹ represent NH(CH₂)ₚOH.

4. Derivative according to claim 3, characterized in that all the R¹ are identical.

5. Derivative according to any one of the claims 1 to 4, characterized in that p is equal to 2.

6. Derivative according to any one of the claims 1 to 5, characterized in that n is equal to 6.

7. Process for the preparation of a cyclodextrin derivative according to claim 2, characterized in that a tosyl derivative of cyclodextrin of formula: 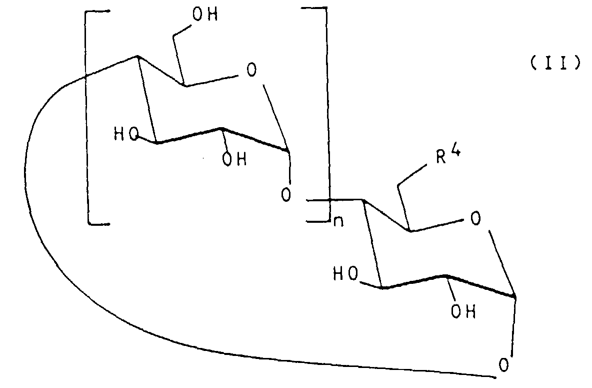 in which R⁴ represents the tosyl group and n is equal to 5, 6 or 7, is reacted with a compound of formula NH₂(CH)₂)ₚOH with p having the meaning given in claim 1.

8. Process for the preparation of a cyclodextrin derivative according to either of the claims 3 and 4, characterized in that an iodocyclodextrin derivative of formula: 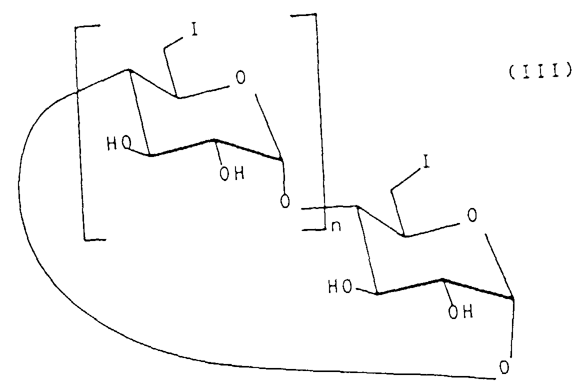 in which n is equal to 5, 6 or 7, is reacted with at least one compound of formula NH₂(CH₂)ₚOH in which p has the meaning given in claim 1.

9. Process for solubilizing in an aqueous medium a hydrophobic chemical compound, characterized in that it consists of combining the hydrophobic chemical compound with a cyclodextrin derivative according to any one of the claims 1 to 6, for forming therewith a water-soluble inclusion complex.

10. Process according to claim 9, characterized in that the hydrophobic compound is a pharmaceutically active molecule.

11. Inclusion complex of a cyclodextrin derivative according to any one of the claims 1 to 6 with a hydrophobic chemical compound.

12. Complex according to claim 11, characterized in that the chemical compound is a pharmaceutically active molecule.

13. Complex according to claim 12, characterized in that the pharmaceutically active molecule is prednisolone.

14. Pharmaceutical composition, characterized in that it comprises an inclusion complex of a cyclodextrin derivative according to any one of the claims 1 to 6 and a pharmaceutically active molecule with a pharmaceutically acceptable vehicle.
